# FASCICULE DE BREVET EUROPEEN

(11) **EP 1 256 330 B1**
(45) Date de publication et mention de la délivrance du brevet: **30.08.2006**
(21) Numéro de dépôt: 02291137.4
(22) Date de dépôt: 06.05.2002
(51) Int. Cl.: A61F 13/36

(54) **Pansement à âme de fibres en alginate entourée d'une enveloppe**
Wundverband mit einem Alginatfaserkern, umgeben von einer Ummantelung
Wound dressing with analginate fibre core wrapped by an envelop

(30) Priorité: 11.05.2001 FR 0106252
(43) Date de publication de la demande: 13.11.2002
(73) Titulaire: Les Laboratoires Brothier, 92000 Nanterre (FR)
(72) Inventeur: Maingault, Philippe, 49700 Douai-la-Fontaine (FR); Barikosky, Michel, 75007 Paris (FR)
(74) Mandataire: Bloch, Gérard

(56) Documents cités:
- WO-A-93/14724
- DE-A- 3 500 842
- US-A- 4 095 542
- US-A- 5 688 260
- US-A- 5 716 337
- US-A- 5 968 026

## Description

L'invention concerne un produit de pansement pour plaie à liquide biologique, comprenant une âme de fibres d'alginate de métal entourée d'une enveloppe de maintien de structure perméable au liquide biologique et biocompatible avec la plaie.

Un tel produit de pansement est notamment connu par EP 0 624 082.

Le produit de pansement en question peut être utilisé pour des plaies humides, notamment profondes, telles que des cavités qui exsudent, comme les escarres ou autres plaies post-opératoires. On peut aussi l'utiliser en chirurgie endonasale, dans les voies aériennes supérieures des fosses nasales.

Comme alginate de métal, on considère fréquemment un alginate d'un métal choisi dans la famille des métaux multivalents, à l'exception du magnésium, et plus particulièrement l'alginate de calcium.

Une plaie provoque une perte de substance, ou de liquide biologique (sang ou exsudat). Appliqué dans une plaie, le pansement commence par absorber le liquide biologique qui suinte, ou qui exsude, les molécules d'eau du liquide s'intercalant entre les macromolécules de l'alginate. Une fois gonflé par absorption, le pansement est soumis à une gélification par échange ionique. Dans le cas de fibres d'alginate de calcium, elles cèdent des ions Ca²⁺ au liquide biologique qui leur cède des ions Na⁺. Au fur et à mesure que s'établit l'équilibre entre le calcium et le sodium, les fibres d'alginate perdent en partie leur structure cristalline. La gélification du pansement entraîne l'assèchement de la plaie et évite qu'il n'adhère aux tissus sous-jacents.

Malgré la gélification, grâce à l'enveloppe de maintien, le produit de pansement reste doté d'une bonne cohésion mécanique et peut relativement facilement être retiré d'une plaie de façon indolore.

Toutefois, le produit de pansement de l'art antérieur introduit ci-dessus n'est pas facile à fabriquer.

L'invention de la présente demande vise à en proposer un qui le soit.

A cet effet, l'invention concerne un produit de pansement du type défini ci-dessus, caractérisé par le fait que l'âme et l'enveloppe se prolongent l'une l'autre en une nappe continue de fibres d'alginate et en ménageant entre elles des lumières de drainage.

Formé d'une unique nappe de fibres, le produit de pansement est naturellement facile à réaliser.

On remarquera que du fait de la structure unitaire du produit de l'invention, l'âme assure également une fonction de support ou de renfort de l'enveloppe qui l'empêche de subir un aplatissement, un affaissement qui réduirait les lumières de drainage. Cela est particulièrement avantageux dans le traitement des cavités, avant l'imprégnation des fibres.

La nappe de fibres peut être une nappe non tissée, tissée, voire tricotée.

On soulignera que les tampons hygiéniques féminins, du type décrit par exemple dans US 4816100, sont aussi constitués d'une nappe continue d'âme-enveloppe. Toutefois, l'enveloppe ne ménage aucune lumière autour de l'âme puisque la fonction du tampon n'est pas d'offrir de bonnes capacités de drainage d'exsudats mais, au contraire, de subir une expansion, de surcroît conique pour assurer convenablement l'obturation de la cavité.

En fait, l'âme et l'enveloppe de ces tampons n'assurent absolument pas les fonctions de soutiens mutuels comme celles du produit de l'invention, l'âme pour l'enveloppe, d'abord, l'enveloppe pour l'âme, ensuite.

Dans la forme de réalisation préférée du produit de l'invention, l'enveloppe est constituée d'une gaine tubulaire dont les bordures adjacentes aux lisières sont repliées vers l'intérieur pour constituer l'âme.

Avantageusement, l'âme s'étend au moins sur sensiblement le quart du diamètre de l'enveloppe, c'est-à-dire le quart de la dimension transversale de la gaine.

De préférence, les bordures de la gaine-enveloppe du produit de pansement de l'invention formant l'âme sont surjetées, c'est-à-dire réunies l'une à l'autre par une couture en surjet. En d'autres termes encore, la gaine-enveloppe est surjetée.

De préférence encore, le surjet de couture se prolonge longitudinalement au-delà de la gaine enveloppe et de l'âme pour constituer un cordon (chaînette) de tirage pour l'extraction du produit de pansement après usage, et de sécurité, pour empêcher le produit de pansement de migrer pendant usage. De préférence toujours, le surjet de couture comporte deux fils.

L'invention sera mieux comprise à l'aide de la description suivante du produit de pansement et de l'équipement permettant de le fabriquer, en référence au dessin annexé, sur lequel :
- la figure 1 est une vue en perspective du produit de pansement ;
- la figure 2 est une vue en perspective de dessus d'une partie de l'équipement de mise en forme de la nappe de fibres en un tube ;
- la figure 3 est une vue en perspective interne de la filière de mise en forme ;
- la figure 4 est une vue en perspective de dessous de l'équipement de mise en forme et de la surjeteuse, du côté opposé à celui de la figure 2 et
- la figure 5 est une vue en perspective de dessus de la surjeteuse.

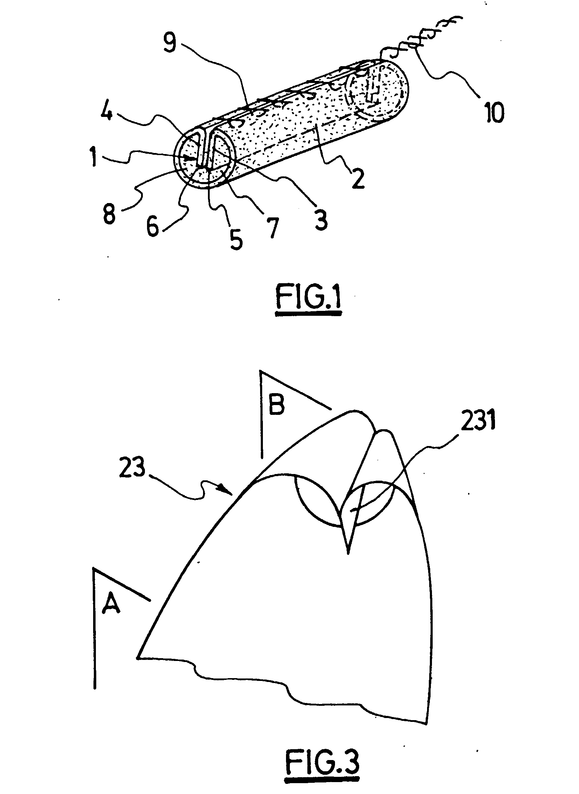

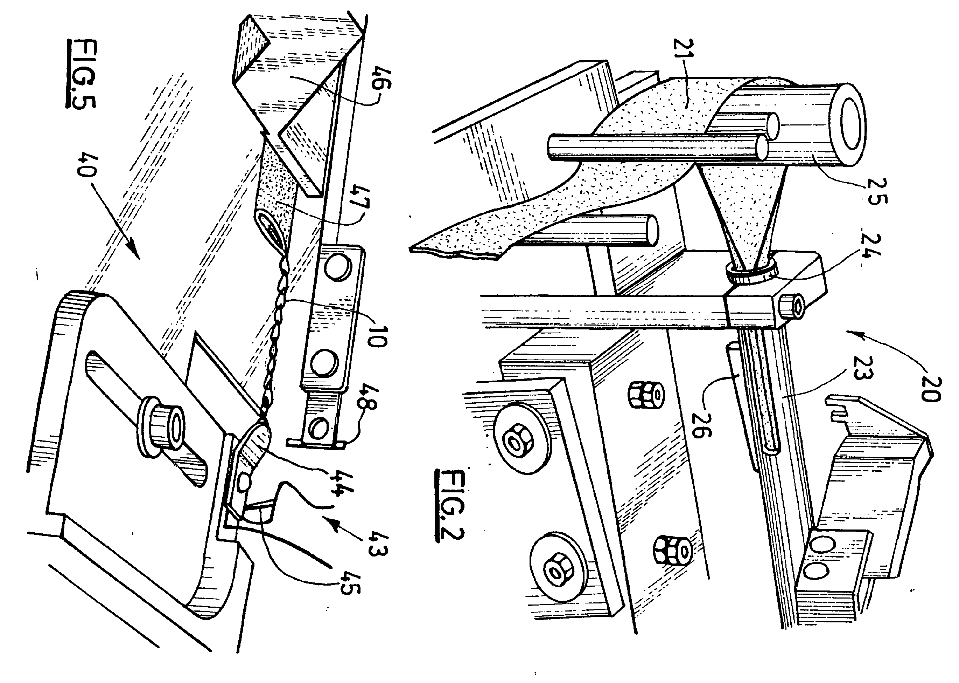

En référence à la figure 1, le produit de pansement est constitué d'une nappe de fibres d'alginate ici non tissée, enroulée, repliée et surjetée.

Ainsi, le pansement comporte une âme 1 et une enveloppe 2 se prolongeant l'une l'autre en une nappe continue. L'âme 1 est constituée par les bordures 3, 4, adjacentes aux lisières 5, 6, qui ont été repliées vers l'intérieur après enroulement sur elle-même de la nappe pour former la gaine-enveloppe ici tubulaire. Deux lumières de drainage 7, 8 s'étendent longitudinalement de part et d'autre de l'âme 1, entre l'âme 1 et l'enveloppe 2. La gaine-enveloppe 2 étant ici sensiblement tubulaire, l'âme 1 s'étend sensiblement dans un plan axial, sur deux tiers du diamètre transversal de l'enveloppe.

La gaine-enveloppe 2 est surjetée, c'est-à-dire que les bordures 3, 4 sont réunies l'une à l'autre par une couture 9 en surjet, le surjet de couture 9 s'étendant longitudinalement au-delà de la gaine-enveloppe 2 en un cordon ou chaînette de tirage et de sécurité 10.

Pour fixer les idées, pour réaliser un pansement avec une gaine-enveloppe d'environ 5 mm de diamètre, la demanderesse est partie d'une laize de 22 mm de large, pour une gaine-enveloppe de 10 mm de diamètre, d'une laize de 46 mm de large. Le pansement peut être réalisé dans une longueur approximative de 40 à 200 mm, avec un cordon de 40 à 100 mm de longueur environ.

Le procédé de fabrication du pansement va maintenant être abordé. La fabrication s'effectue à l'aide d'un équipement de mise en forme 20 et d'une surjeteuse 40.

La mise en forme consiste, à partir d'une laize 21, à former un produit qui progressivement va prendre la forme d'un tube ouvert, d'un tube à bordures longitudinales rentrées et repliées vers l'intérieur puis d'un tel tube écrasé 22, avant d'être coupé et entraîné dans la surjeteuse.

La mise en forme s'effectue dans un guide ou filière 23, la laize étant déjà presque totalement enroulée sur elle-même à l'entrée 24 de la filière. La nappe enroulée avance dans la filière 23 sous l'action d'un rouleau rotatif d'avance 25, extérieur à la filière et d'axe orthogonal à celui de la filière, et d'un jet d'air comprimé introduit tangentiellement dans la filière, par une buse latérale 26, pour " lubrifier " la filière et faciliter l'avance de la nappe en cours de formation (figure 2).

En référence à la figure 3, on voit bien la forme axialement évolutive de la paroi intérieure de la filière 23, depuis une section A de tube ouvert jusqu'à une section circulaire B de tube refermé sur lui-même avec bordures repliées vers l'intérieur, grâce à la portion de paroi médiane d'extrémité 231 en forme de double extrados.

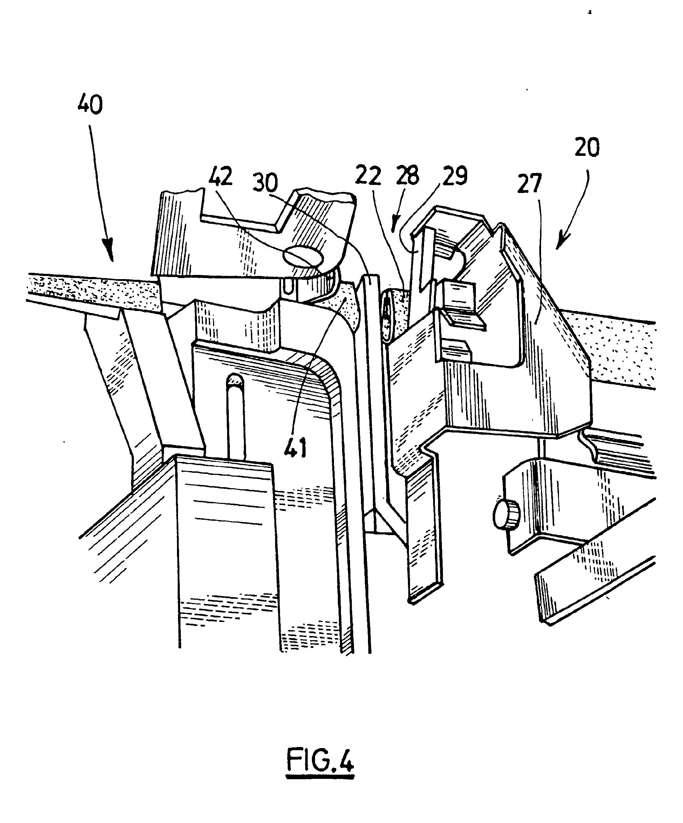

La nappe tubulaire 22 sortant de la filière 23 est traitée et écrasée par une pince 27 avant d'être saisie dans la surjeteuse 40 et coupée par un ensemble de coupe 28 à couteau 29 et contre-couteau 30 entre la pince 27 et la surjeteuse 40 (figure 4).

La nappe tubulaire 41 préformée avant couture dans la surjeteuse y est entraînée par un pied de traction 42 (figure 4) jusqu'à la station de couture à surjet 43, avec le pied de couture 44 et l'aiguille 45 (figure 5).

Sous l'action d'un pied presseur et de translation 46, le produit de pansement 47 ainsi fabriqué est tracté sur la longueur du cordon de tirage et de sécurité qu'on veut réaliser avant que ce cordon 10 ne soit coupé à la bonne longueur par un couteau de coupe 48.

## Revendications

1. Produit de pansement pour plaie à liquide biologique, comprenant une âme (1) de fibres d'alginate de métal entourée d'une enveloppe (2) de maintien de structure perméable au liquide biologique et biocompatible avec la plaie, **caractérisé par le fait que** l'âme (1) et l'enveloppe (2) se prolongent l'une l'autre en une nappe continue de fibres d'alginate et en ménageant entre elles des lumières de drainage (7, 8).

2. Produit selon la revendication 1, dans lequel la nappe de fibres d'alginate est choisie dans le groupe des nappes non tissées, tissées et tricotées.

3. Produit selon l'une des revendications 1 et 2, dans lequel l'enveloppe est constituée d'une gaine tubulaire (2) dont les bordures (3, 4) adjacentes aux lisières (5, 6) sont repliées vers l'intérieur pour constituer l'âme (1).

4. Produit selon l'une des revendications 1 à 3, dans lequel l'âme (1) s'étend au moins sur sensiblement le quart de la dimension transversale de l'enveloppe (2).

5. Produit selon l'une des revendications 3 et 4, dans lequel les bordures (3, 4) de l'enveloppe (2) sont surjetées.

6. Produit selon la revendication 5, dans lequel il est prévu un surjet de couture (9) qui se prolonge au-delà de la gaine (2) et de l'âme (1) en un cordon de tirage et de sécurité (10).

## Claims

1. Biological fluid wound dressing product comprising a core (1) of metal alginate fibres surrounded by a structure holding wrapper (2) permeable to the biological fluid and biocompatible with the wound, **characterised in that** the core (1) and the wrapper (2) extend mutually as a continuous sheet of alginate fibres and have drainage holes (7, 8) between them.

2. Product according to Claim 1 wherein the sheet of alginate fibres is selected from the group of non-woven, woven and knitted sheets.

3. Product according to one of the Claims 1 and 2, wherein the wrapper consists of a tubular sheath (2), the edges of which (3, 4) adjacent to the selvedges (5, 6) are folded towards the inside to form the core (1).

4. Product according to one of the Claims 1 to 3, wherein the core (1) extends at least more or less over a quarter of the transverse dimension of the wrapper (2).

5. Product according to one of the Claims 3 and 4, wherein the edges (3, 4) of the wrapper (2) are overcast.

6. Product according to Claim 5, wherein an overcast seam (9) is provided and it extends beyond the sheath (2) and the core (1) as a pull and safety cord (10).

## Patentansprüche

1. Wundverbandsprodukt für Wunden mit biologischer Flüssigkeit, mit einem Kern (1) aus Metallalginatfasern, der von einer Einfassung (2) umgeben ist, die die Struktur hält und die die biologische Flüssigkeit durchlässt und mit der Wunde verträglich ist, **dadurch gekennzeichnet, daß** sich der Kern (1) und die Einfassung (2) gegenseitig verlängern als durchgehende Schicht aus Alginatfasern und unter Bildung von Drainageöffnungen (7, 8) zwischen einander.

2. Produkt nach Anspruch 1, wobei die Schicht aus Alginatfasern ausgewählt ist aus der Gruppe von nichtgewebten, gewebten und gestrickten Schichten.

3. Produkt nach einem der Ansprüche 1 und 2, wobei die Einfassung aus einem rohrförmigen Mantel (2) gebildet ist, dessen den Kanten (5, 6) benachbarte Ränder nach innen umgebogen sind, um den Kern (1) zu bilden.

4. Produkt nach einem der Ansprüche 1 bis 3, wobei sich der Kern (1) wenigstens über im wesentlichen ein Viertel der transversalen Abmessung der Einfassung (2) erstreckt.

5. Produkt nach einem der Ansprüche 3 und 4, wobei die Ränder (3, 4) der Einfassung (2) überwendlich genäht sind.

6. Produkt nach Anspruch 5, wobei eine Überwendlichnaht (9) vorgesehen ist, die sich über den Mantel (2) und den Kern (1) hinaus als Zug- und Sicherheitsschnur (10) erstreckt.
